# EUROPÄISCHE PATENTSCHRIFT

(11) **EP 2 760 809 B1**
(45) Veröffentlichungstag und Bekanntmachung des Hinweises auf die Patenterteilung: **20.02.2019**
(21) Anmeldenummer: 12746374.3
(22) Anmeldetag: 16.08.2012
(51) Int. Cl.: C07C 1/20, C07C 11/06, B01J 8/04

(54) **VERFAHREN UND ANLAGE ZUR HERSTELLUNG VON OLEFINEN AUS DIMETHYLETHER**
METHOD AND SYSTEM FOR PRODUCING OLEFINS FROM DIMETHYL ETHER
PROCÉDÉ ET INSTALLATION DESTINÉS À LA PRÉPARATION D'OLÉFINES À PARTIR D'ÉTHER DIMÉTHYLIQUE

(30) Priorität: 27.09.2011 DE 102011114367
(43) Veröffentlichungstag der Anmeldung: 06.08.2014
(73) Patentinhaber: Air Liquide Global E&C Solutions Germany GmbH, 60439 Frankfurt am Main (DE)
(72) Erfinder: BIRKE, Gerhard, 60389 Frankfurt (DE); AHLERS, Bernd, 63128 Dietzenbach (DE); KÖMPEL, Harald, 63263 Neu-Isenburg (DE); LIEBNER, Waldemar, 61440 Oberursel (DE)
(74) Vertreter: Keil & Schaafhausen Patent- und Rechtsanwälte PartGmbB
(86) Internationale Anmeldenummer: PCT/EP2012/066022
(87) Internationale Veröffentlichungsnummer: WO 2013/045169

(56) Entgegenhaltungen:
- EP-A1- 2 058 290
- DE-A1-102005 015 923
- US-A1- 2006 063 956

## Beschreibung

Die Erfindung betrifft die Herstellung von Olefinen aus Dimethylether.

Kurzkettige Olefine, insbesondere Propylen (Propen), gehören zu den wichtigsten Grundstoffen der chemischen Industrie. Ausgehend von diesen ungesättigten Verbindungen mit einer kurzen Kettenlänge lassen sich Moleküle mit längerkettigem Kohlenstoffgerüst und zusätzlichen Funktionalisierungen aufbauen.

Als Quelle für kurzkettige Olefine diente in der Vergangenheit vor allem das Steam-Cracking, d.h. die thermische Spaltung bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen)- oder auch MTO- (Methanol-to-Olefin)-Verfahren zur Herstellung von Propylen und anderen kurzkettigen Olefinen gehen von Methanol als Ausgangsstoff aus. In diesen heterogen katalysierten Verfahren wird aus Methanol zunächst teilweise das Zwischenprodukt Dimethylether (DME) und aus einer Mischung von Methanol / Dimethylether dann eine Mischung aus Ethylen und Propylen gebildet.

Im klassischen MTP-Verfahren ist Reinmethanol Ausgangsstoff für die Umsetzung; dieses wird zunächst destillativ aus Rohmethanol gewonnen. Sodann wird Methanol unter hohem Energieaufwand verdampft und im einfachen Durchlauf zu dem DME-Reaktor geführt, in dem es zu Dimethylether und Wasser umgesetzt wird. Das so entstehende Produktgemisch enthält Methanol/DME in einem Verhältnis von ca. 30:70 Gew.-% und wird gekühlt und teilkondensiert.

Das wasserreiche Kondensat aus dem Produkt des DME-Reaktors wird zusammen mit gasförmigem Dimethylether / Methanol auf die einzelnen Stufen des Reaktors aufgegeben, wo Methanol und Dimethylether zu hauptsächlich olefinischen Kohlenwasserstoffen umgesetzt werden. Das bei dieser Umsetzung entstehende Produktgemisch muss anschließend einer weiteren Aufreinigung unterzogen werden.

Die DE 11 2006 002 599 T5 beschreibt beispielsweise, wie durch entsprechende Prozessparameter vor allem Ethylen gewonnen und durch Schaltung von Rektifikationseinrichtungen effizient aufgereinigt werden kann.

Ursache für das Entstehen eines Produktgemisches ist vor allem ein deutlich ausgeprägtes Wärmeprofil über die einzelnen Katalysatorstufen im Olefinreaktor. Es wird versucht, dem entgegenzuwirken, indem die Edukte teilweise in flüssiger Form aufgegeben werden. Als Folge der Verdampfung von Kondensat wird der Gasstrom vor Eintritt in das nächste Katalysatorbett gekühlt. Dennoch bleibt die Reaktionsführung aufgrund der Exothermie der im Reaktor ablaufenden Reaktionen, insbesondere der von Methanol ausgehenden Reaktionen, schwierig. Es kommt zu einer starken Wärmetönung innerhalb der einzelnen Katalysatorstufen und zu den damit verbundenen Nachteilen, insbesondere zu Katalysatorschädigungen und einer Abnahme der Selektivität bezogen auf das erwünschte Produkt.

Aus der DE 695 199 99 T2 ist bekannt, dass in einen Reaktor zur Erzeugung von Olefinen ein Gemisch aus Alkoxyverbindungen, wie bspw. Methanol und Dimethylether, eingebracht werden kann, wobei diesem Gemisch ein Verdünnungsgas zugemischt wird. Durch das Verdünnungsgas kann erreicht werden, dass die Temperatur des aufgegebenen Gasstroms zwischen rund 340 und 400 °C liegt und die spezifische Wärmekapazität des entstehenden Gasstroms sich so verändert, dass die Temperaturerhöhung innerhalb des Gasstroms geringer als 100 °C ist. Dadurch wird zwar vermieden, dass sich lokal Wärmezonen ausbilden, die den Katalysator schädigen und in denen unerwünschte Reaktionen ablaufen, gleichzeitig liegt diese Temperatur jedoch deutlich unterhalb der optimalen Betriebstemperatur. Um einen vergleichbaren Umsatz zu erzielen, muss die Verweilzeit erhöht werden. Damit sinkt der Umsatz pro Zeiteinheit und somit die Verfahrenseffizienz.

Das im klassischen MTP-Prozess im DME-Zwischenprodukt enthaltene Methanol wirkt sich insofern nachteilig aus, als die Umsetzung im Olefin-Reaktor durch eine stark exotherme Reaktion erfolgt. Die Wärmetönung des Reaktors ist dabei beim Einsatz eines Methanol-DME-Gemisches deutlich höher als bei der Verwendung von reinem Dimethylether. Eine Umstellung auf reinen Dimethylether ist jedoch bei konventioneller Ausführung des MTP-Prozesses aufwändig und nicht wirtschaftlich, weil zwei separate Destillationssysteme zur Trennung von Rohmethanol / Reinmethanol sowie von DME / Methanol / H₂O mit hohem Investitionsaufwand und Hilfsmittelverbrauch ausgeführt werden müssen.

Bei einem in der DE 10 2008 058 931 B4 beschriebenen Verfahren wird die Aufreinigung von Dimethylether vorteilhaft mit der Reinigung des Rohmethanols verknüpft. In einem Reaktor wird durch eine heterogen katalysierte Reaktion ein Gemisch von Methanol, Dimethylether und Wasser erzeugt, welches im Hinblick auf die Abtrennung der einzelnen Bestandteile aufbereitet werden muss. Dazu wird das Gemisch aus dem Reaktor einer ersten Destillationskolonne zugeführt, in der ein Gemisch aus Methanol und Dimethylether von dem im Wesentlichen aus Wasser bestehenden Sumpfprodukt abgetrennt wird. Das Methanol-DME-Gemisch wird einer zweiten Destillationskolonne zugeführt, in der über Kopf der aufgereinigte Dimethylether abgezogen wird. Das zum größten Teil aus Methanol bestehende Sumpfprodukt wird einer dritten Destillationskolonne zugeführt, welche gleichzeitig mit dem Ausgangsstoff Rohmethanol gespeist wird. Das in dieser dritten Kolonne gereinigte Methanol kann als Edukt für den Reaktor zur Umsetzung von Dimethylether verwendet werden. Durch dieses Verfahren kann also die Methanoldestillation und die Aufreinigung des Dimethylethers in einem Prozess zusammengefasst werden.

Das aus der DE 10 2008 058 931 B4 bekannte Verfahren erlaubt es, Dimethylether als einzige Einsatzkomponente für die Umsetzung zu Olefinen zu verwenden und gleichzeitig die Wirtschaftlichkeit des Verfahrens zu gewährleisten.

Die DE 10 2005 015923 A1 beschreibt ein Verfahren zur Herstellung von C₂ bis C₄ Olefinen. Dazu wird ein Oxygenat und Wasserdampf enthaltender Einsatzstrom in einem Reaktor ausgeformte Festbettzonen geleitet. Anschließend wir das C₂ bis C₃ Olefine und inerte Gaskomponenten enthaltende Produkt einer Aufreinigung zugeführt. Innerhalb dieser Aufreinigung wird in einer Trennanlage ein sogenannter Zusatzstrom gewonnen. Dieser Zusatzstrom wird in drei Zusatzteilströme aufgeteilt und anteilig über Leitungen den verschiedenen Festbetten innerhalb des Reaktors zugeführt. Über diesen Zusatzstrom ist es möglich, die Temperatur in dem Festbett teilweise zu steuern.

Die EP 2 058 290 A1 beschreibt ein Verfahren zur Herstellung von Propylen, bei dem in einem oxygenathaltigen Strom auch C₄ und/oder C₅-Olefine in den Reaktor eingebracht werden. Diese Olefine stammen aus der Aufreinigung des im Reaktor erzeugten Produktes, wobei keine unterschiedliche lokale Einbringung dieser Rückführungsstroms mittels Leitung vorgesehen ist.

Die US 2006/063956 A1 beschreibt ebenfalls ein Verfahren zur Herstellung von Propylen aus einem oxygenathaltigen Eduktstrom, wobei auch hier der oxygenathaltigen Eduktstrom über eine Leitung verschiedenen Festbetten zugeführt wird. Aus den in dieser Reaktion entstehenden Gemisch wird ein kohlenstoffhaltiger Strom in einer Aufreinigungsvorrichtung abgetrennt und über eine Leitung wenigstens teilweise zurückgeführt. Die Rückführung erfolgt so, dass verschiedene Teilströme über Leitungen abgezweigt werden, so dass jeweils vor jedem Festbettreatktor einer der Teilströme eindosiert werden kann.

Ausgehend davon liegt der vorliegenden Erfindung die Aufgabe zugrunde, aufgereinigten Dimethylether in vorzugsweise mehreren Katalysatorstufen in einem heterogen katalysierten Verfahren zu Olefinen umzusetzen, wobei das Temperaturprofil über die Katalysatorstufen möglichst flach ausgeprägt, jedoch nah an der optimalen Betriebstemperatur sein soll.

Diese Aufgabe wird erfindungsgemäß mit den Merkmalen des Anspruchs 1 gelöst. Der gasförmige Dimethylether in einer Reinheit von 70 Gew.-% - 100 Gew.-%, vorzugsweise 85 Gew.-% - 99 Gew.-%, weiter bevorzugt einer Reinheit von wenigstens 95 Gew.-% und besonders bevorzugt von wenigstens 97,5 Gew.-% wird in einer Mischung mit Recyclegas auf eine Katalysatorstufe aufgegeben. Dadurch ist eine optimierte Reaktionsführung möglich, insbesondere ist es nicht mehr notwendig, ein Wasser/ Methanol-Gemisch in flüssiger Form vor den einzelnen Katalysatorstufen einzubringen und den Kühlungseffekt der Verdampfung zu nutzen.

Weiterhin hat es sich als vorteilhaft herausgestellt, innerhalb eines Reaktors mehrere Katalysatorstufen hintereinander zu schalten und diese so miteinander zu verbinden, dass Produktgas aus einer Katalysatorstufe in eine andere Katalysatorstufe gespeist wird. Dies hat den Vorteil, dass unverbrauchtes Edukt aus dem Produktgemisch der vorgeschalteten Katalysatorstufe in der nachgeschalteten Katalysatorstufe umgesetzt werden kann, wodurch die Ausbeute des Prozesses weiter erhöht werden kann.

Auf die erste Katalysatorstufe werden Recyclegas und Wasserdampf aufgegeben. Das Recyclegas enthält vorzugsweise olefinische sowie paraffinische und/oder aromatische Kohlenwasserstoffe.

Der Wasserdampf, der dem Recyclegas vor der Erhitzung auf Reaktionstemperatur zugegeben wird, dient als Wärmeträger und Moderator und reduziert eine Verkokung bei hohen Temperaturen und damit auch eine vorzeitige Deaktivierung des Katalysators. Die Zugabe von Wasserdampf kann dabei entweder direkt in die Katalysatorstufe oder als Mischstrom mit dem Recyclegas erfolgen. Die Zugabe eines einzigen Stroms erleichtert die Verfahrensführung und verläuft besonders günstig, wenn das Recyclegas unter Zugabe von bis zu 60 Gew.-% Wasserdampf bezogen auf das Recyclegas in einer Unterfeuerungseinheit auf die Reaktoreintrittstemperatur erhitzt wird.

Um in jeder Katalysatorstufe möglichst homogene Bedingungen einstellen zu können, empfiehlt es sich zudem, diesen Strom aus Recyclegas und Wasserdampf mit möglichst schonend vorerhitztem Dimethylether zu vermischen und so nur einen Gesamtstrom auf die erste Katalysatorstufe aufgegeben. Der Anteil von Dimethylether im Verhältnis zum Recyclegas am Eintritt in die erste Katalysatorstufe liegt bevorzugt im Bereich von 5-20 Gew.-%, besonders bevorzugt bei 10 bis 15 Gew.-%.

Es hat sich als günstig herausgestellt, dass das Verhältnis der auf die erste Katalysatorstufe aufgegebenen Gasströme so eingestellt wird, dass der Anteil des Dimethylethers im Verhältnis zum Recyclegas bei Eintritt in die Katalysatorstufe 2 bis 20 Gew.-% und bevorzugt 5-15 Gew.-% und der Anteil des Wasserdampfes am Gesamtstrom bei Eintritt in die Katalysatorstufe maximal 50 Gew.-% beträgt.

Auf die zweite und jede weitere Katalysatorstufe wird das Produktgas der vorhergehenden Stufe und eine Mischung von Dimethylether und Recyclegas im Verhältnis von 10 - 200 Gew.-%, bevorzugt 15-50 Gew.-% Recyclegas im Verhältnis zum Dimethylether aufgegeben. Auch hier kann grundsätzlich eine separate Aufgabe der einzelnen Ströme erfolgen.

Indem man das Verhältnis von Dimethylether und Recyclegas zueinander und die Eintrittstemperaturen eines oder beider Ströme (fein)regelt, kann erreicht werden, dass die Eintrittstemperatur in eine Katalysatorstufe zwischen 420 und 500 °C, bevorzugt 440 und 490 °C, und/oder die Austrittstemperatur aus der Katalysatorstufe zwischen 450 und 530 °C, bevorzugt 460 und 520 °C, liegt. Bei diesen Temperaturen kommt es zu einem nahezu vollständigen Umsatz des Dimethylethers, ohne dass dies zu einer verringerten Selektivität hinsichtlich der erwünschten Produkte, vor allem Ethylen und Propylen, führen würde. Diese Temperaturen liegen deutlich über denen, die bei herkömmlichen Verfahren (Eintrittstemperatur ca. 400 °C) praktiziert werden.

Die Eintrittstemperatur ergibt sich als Mischtemperatur des Dimethylethers mit dem Recyclegas und dem Produktstrom aus einer vorgeschalteten Katalysatorstufe. Die Austrittstemperatur ergibt sich aus der Eintrittstemperatur in eine Katalysatorstufe sowie der Temperaturerhöhung als Folge exothermer Reaktionen, wobei diese Exothermie teilweise durch Endothermie anderer Reaktionen ausgeglichen werden kann.

Vorteilhaft ist es zudem, wenn das Verhältnis von Dimethylether und Recyclegas so geregelt oder gesteuert wird, dass die Temperatur innerhalb eines Katalysatorbetts um wenigstens 5 jedoch um maximal 100 °C, bevorzugt 40 °C ansteigt. Dies hat den Vorteil, dass die Bildung von sog. Hot-Spots, d.h. lokalen Erwärmungen innerhalb der Katalysatorstufe, zuverlässig vermieden werden kann. Dadurch verbessert sich die Standzeit des Katalysators, da dieser nicht aufgrund von lokalen Erwärmungen vorzeitig altert.

Günstig ist es insbesondere auch, die Beschickung jeder einzelnen Katalysatorstufe mit Dimethylether und Recyclegas separat zu regeln, da so innerhalb jeder Katalysatorstufe Bedingungen eingestellt werden können, die unter Berücksichtigung des eingespeisten Produktgasstroms aus einer vorgeschalteten Katalysatorstufe zu einer Ausbeutemaximierung führen.

Das Recyclegas wird mit unterschiedlichen Zusammensetzungen verwendet, wodurch weitere Möglichkeiten zur Prozessoptimierung innerhalb der einzelnen Katalysatorstufen gegeben sind. Beispielsweise kann das Recyclegas auch Methanol enthalten und die mit der Methanol-Abreaktion verbundene Wärmetönung kann in einer Katalysatorstufe eingebracht werden, wo dies erwünscht ist oder mit dem geringsten Nachteil einhergeht, z. B. bei der ersten Katalysatorstufe.

Gemäß einer besonders bevorzugten Ausgestaltung der Erfindung wird Recyclegas mit einer abweichenden Zusammensetzung auf die erste Katalysatorstufe aufgegeben, während alle nachfolgenden Katalysatorstufen mit Recyclegas gleicher Zusammensetzung beschickt werden. Günstig ist es hierbei auch, Recyclegas mit einem besonders hohen Olefinanteil, insbesondere mit Kettenlängen C₄ bis C₈ zu verwenden. Da diese Olefine im Reaktor in einer endothermen Reaktion zu Propylen reagieren, dienen sie gleichzeitig der Kühlung des Systems und der Erhöhung der Ausbeute an Zielprodukt. Insgesamt soll die Eindosierung in die einzelnen Katalysatorstufen so erfolgen, dass durch die Temperatur- und Reaktionsführung die Gesamtausbeute an Propylen über alle Katalysatorstufen hinweg maximiert wird. Die Verfahrenseffizienz lässt sich dadurch weiter steigern, dass die Eindosierung in die einzelnen Katalysatorstufen so erfolgt, wie es der Alterungszustand des jeweiligen Katalysators erfordert. Dies bedeutet insbesondere, dass bei einem in Folge von Alterungsprozessen weniger aktiven Katalysator mit einer höheren Eintrittstemperatur gearbeitet werden kann. Dies kann beispielsweise durch Erhöhung des Methanolgehalts im Eintrittsstrom erreicht werden.

Das Verfahren lässt sich insbesondere mit einem Druck am Eintritt des Reaktors von 0.8 - 5.0 bar (a), vorzugsweise 1.5-3.0 bar (a) mit hoher Ausbeute betreiben.

Das Verfahren lässt sich zudem insbesondere dann mit guten Katalysatorstandzeiten und einer hohen Ausbeute an kurzkettigen Olefinen wie Propylen betreiben, wenn als Katalysator ein formselektives Zeolithmaterial, vorzugsweise ein Alumosilikat vom Pentasiltyp ZSM-5, verwendet wird.

Die Wirtschaftlichkeit des Verfahrens kann überdies dadurch erhöht werden, dass das Recyclegas innerhalb der Aufreinigung gewonnen wird, der die aus Dimethylether gewonnenen Olefine unterzogen werden. Somit können anderenfalls im Prozess entstehende Abwässer minimiert werden, bzw. darin enthaltene Kohlenstofffraktionen teilweise durch ein erneutes Durchlaufen der Katalysatorstufe noch zu Wertprodukt umgesetzt werden. Bevorzugt lassen sich dabei Ströme mit einem Anteil von 10-70 Gew.-%, bevorzugt 20-40 Gew-% C₂- und/oder C₄-C₈ -Olefinen als Recyclegas verwenden.

Insbesondere fällt ein als Recyclegas geeigneter Gasstrom dann an, wenn in einer solchen Aufbereitungseinheit die erzeugten Kohlenwasserstoffe mit Methanol als Waschmittel aufgereinigt werden. Das nach seiner Verwendung als Waschmittel verwendete Methanol kann in einer Destillationseinheit aufbereitet werden. Das Kopfprodukt dieser Destillationseinheit kann dann wenigstens teilweise als Recyclegas direkt zum Reaktor, vorzugsweise als Recyclegas zu wenigstens einer der ersten Katalysatorstufe nachgeschalteten Katalysatorstufen, geführt werden.

Vorteilhaft kann auch das Methanol, welches als Sumpfprodukt dieser Destillationskolonne erzeugt wird, weiterverwertet werden, indem es dem DME-Reaktor zugeführt wird. Dies geschieht dadurch, dass dieses Sumpfprodukt als Rückfluss in diejenige Destillation zurückgeführt wird, die auch zur Reinigung des Rohmethanols für die Umsetzung zu DME dient.

Als günstig hat es sich weiterhin herausgestellt, dem erfindungsgemäßen Verfahren ein Verfahren zur Herstellung von reinem Dimethylether aus Rohmethanol vorzuschalten. Dazu wird das in einem ersten Verfahrensschritt erzeugte Rohmethanol einer wenigstens einstufigen Destillationsstufe zugeführt und von dort in einem zweiten Verfahrensschritt innerhalb einer Reaktionsstufe zu Dimethylether umgesetzt. Anschließend wird der so gewonnene Dimethylether in einer wenigstens einstufigen, bevorzugt wenigstens zweistufigen Destillation aufgereinigt, wobei rückgewonnenes Methanol in diejenige Destillation zurückgeführt wird, die auch zur Reinigung des Rohmethanols dient.

Bevorzugt ist die zweistufige Destillation so ausgestaltet, dass innerhalb eines ersten Reinigungsschrittes Wasser von einem Methanol-Dimethylether-Gemisch getrennt und das Methanol-Dimethylether-Gemisch anschließend in einer zweiten Destillation bezüglich der beiden Hauptkomponenten aufgetrennt wird.

Die Erfindung umfasst weiterhin eine Anlage zur Herstellung von Olefinen aus Dimethylether mit den Merkmalen des Anspruchs 6, die zur Durchführung des erfindungsgemäßen Verfahrens geeignet ist. Eine solche Anlage weist einen Reaktor mit wenigstens zwei Katalysatorstufen auf. In wenigstens eine Katalysatorstufe mündet wenigstens eine Zufuhrleitung für Dimethylether, Recyclegas und Wasserdampf, wobei diese Komponenten vermischt oder einzeln eingespeist werden können. In wenigstens eine nachgeschaltete, mit der ersten Stufe verbundene Katalysatorstufe wird über eine Leitung Recyclegas eingespeist. Zudem wird über diese oder eine zweite Leitung Dimethylether zugeführt. Das jeweils verwendete Recyclegas enthält olefinische, paraffinische und aromatische Kohlenwasserstoffe. Zur Dosierung des Recyclegases im Verhältnis zu dem eingesetzten Dimethylether ist in jeder Zufuhrleitung wenigstens eine Steuer- oder Regelvorrichtung vorgesehen.

Vorzugsweise ist die Regelvorrichtung so ausgestaltet, dass die Temperaturen des Eintrittsstroms in die jeweilige Katalysatorstufe und des entsprechenden Austrittsstroms gemessen und als Regelgrößen für die zu dosierenden Mengen an Recyclegas und Dimethylether verwendet werden, da so der für die Reaktionsführung entscheidende Parameter, die Temperatur, direkt als Regelgröße verwendet werden kann. In Weiterbildung der Erfindung können auch innerhalb des Katalysatorbetts der jeweiligen Katalysatorstufe Temperaturmessstellen vorgesehen und die dort ermittelten Temperaturen als Regelgröße verwendet werden.

Erfindungsgemäß hat es sich bei einer solchen Schaltung von mehreren aufeinander folgenden Katalysatorstufen als günstig herausgestellt, wenn jede Katalysatorstufe mit einer eigenen Zufuhrleitung für Recyclegas und wenigstens einer zugehörigen Steuer- oder Regeleinrichtung ausgestattet ist, da so die Verfahrensbedingungen der einzelnen Katalysatorstufen unabhängig geregelt werden können.

Eine vorteilhafte Ausführung der Erfindung sieht zudem vor, dass aus wenigstens einer Einrichtung zur Aufbereitung der Olefine eine Leitung zu der Zufuhrleitung für das Recyclegas führt.

Wenn die Aufreinigung von Rohmethanol mit der Reinigung des Dimethylether verknüpft werden soll, weist die Anlage erfindungsgemäß eine Destillationseinrichtung zur Aufreinigung von Rohmethanol sowie einen Reaktor zur Umsetzung von Rohmethanol zu Dimethylether auf. Ferner enthält eine solche Anlage wenigstens zwei Destillationseinrichtungen zur Aufreinigung des Dimethylethers, wobei eine der weiteren Destillationseinrichtungen über eine Leitung mit dem Reaktor zur Olefinherstellung verknüpft ist. Zudem führt eine Leitung von einer der weiteren Destillationseinrichtungen zurück zur ersten Destillationseinrichtung zur Aufreinigung von Rohmethanol.

Weitere Merkmale, Vorteile und Anwendungsmöglichkeiten der Erfindung ergeben sich auch aus der nachfolgenden Beschreibung eines Ausführungsbeispiels und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezügen.

Es zeigen:
- Fig. 1: das grundsätzliche Verfahrensschema ohne Olefinreaktor;
- Fig. 2: schematisch eine erfindungsgemäße Ausgestaltung eines Olefinreaktors, der sich an die Anlage gemäß Fig. 1 anschließt.

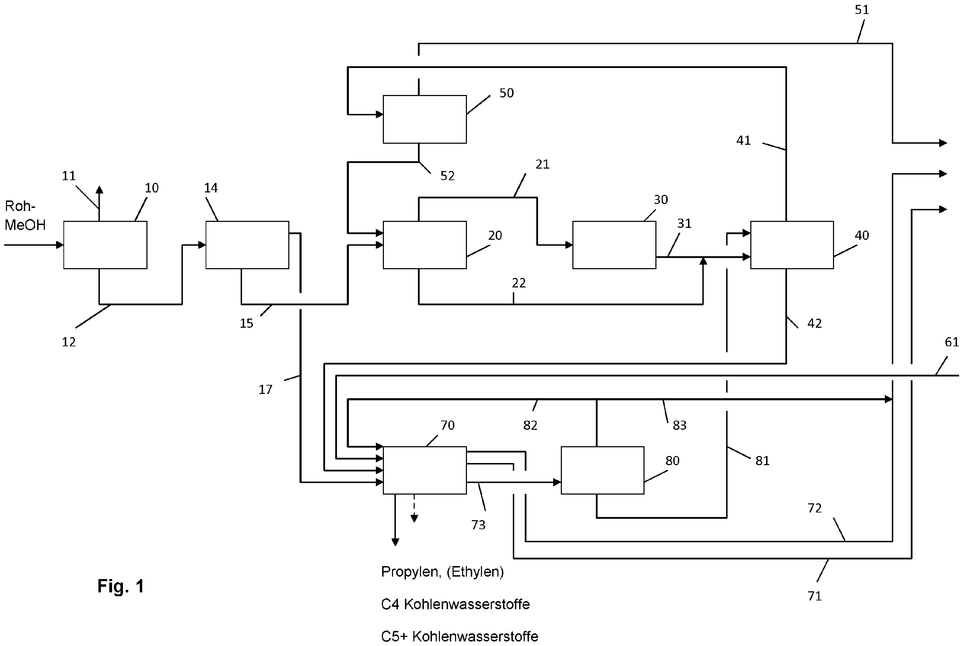

Bei der in Fig. 1 in Form eines Fließdiagramms dargestellten Anlage zur Herstellung von Olefinen aus Rohmethanol wird das Rohmethanol in eine erste Destillations- oder Rektifikationskolonne 10 eingespeist. Die Kolonne weist in üblicher, dem Fachmann bekannter Weise einen Kolonnenkörper, einen Verdampfer im Sumpf der Kolonne und einen Kondensator im Kopf der Kolonne sowie zusätzliche Einbauten wie Böden, Füllkörper und/oder Packungen auf. Weiterhin sind die Kolonnen typischerweise so ausgestattet, dass nur ein kleiner Teil des Kopfkondensats abgezogen und gesammelt wird, während der Großteil wieder als Rückfluss in den Kolonnenkopf eingespeist wird, um die Trennwirkung zu verbessern.

Aus der Rektifikation 10 können über Leitung 11 in Rohmethanol gelöste Gase und Leichtsieder, insbesondere kurzkettige Alkane und Oxygenate, abgezogen werden. Über Leitung 12 wird das als Sumpfprodukt der Kolonne 10 abgezogene Methanol in eine zweite Destillations- oder Rektifikationskolonne 14 eingespeist. Aus dem Sumpf dieser Kolonne 14 wird über Leitung 15 ein Methanol/Wasser Gemisch abgezogen und der Trennvorrichtung 20 zugeführt. Ebenso wird über Leitung 52 das aus Methanol und Spuren DME / Wasser bestehende Sumpfprodukt der Kolonne 50 als Rückfluss auf die Kolonne 20 aufgegeben. Das Kopfprodukt der Kolonne 20 enthält überwiegend Methanol, welches gasförmig über Leitung 21 in den DME-Reaktor 30 gespeist wird. Das im Sumpf angereicherte Wasser wird über Leitung 22 zur Kolonne 40 weitergeleitet.

In der Destillationskolonne 14 wird Reinmethanol über einen in Leitung 17 mündenden Seitenabzug gewonnen. Reinmethanol kann so als Nebenprodukt exportiert werden. Es kann jedoch auch, wie aus der DE 10 2011 014 892 bekannt, als Waschmittel in einer Methanolwaschkolonne verwendet werden. Eine solche, nicht dargestellte Kolonne kann in die Aufreinigungseinrichtung 70 integriert sein und zur Entfernung von DME und weiteren Oxygenaten aus Roh-Propylen verwendet werden. Soll weder Reinmethanol exportiert noch eine Waschkolonne in der Aufbereitungseinheit 70 ausgeführt werden, kann die Kolonne 14 vollständig entfallen. In diesem Falle wird das Sumpfprodukt der Kolonne 10 über Leitung 12 in Leitung 15 und von dort direkt zur Kolonne 20 geführt.

Der in dem Reaktor 30 mittels heterogener Katalyse aus Methanol erzeugte Dimethylether wird über Leitung 31 einer destillativen Aufreinigung in Kolonne 40 zugeführt. In Kolonne 40 wird das Wasser, welches sowohl als Verunreinigung des Rohmethanols schon im Eduktstrom enthalten ist als auch bei der Bildung von Dimethylether gebildet wird, von dem Gemisch aus Methanol und Dimethylether getrennt.

Das im Wesentlichen aus Wasser bestehende Sumpfprodukt der Kolonne 40 wird über Leitung 42 der Aufreinigungseinrichtung 70 zugeführt, die auch einen nicht dargestellten Wasserstripper enthält. Dieses Sumpfprodukt enthält noch zwischen 0,5 und 2 Gew.-% Methanol, die in dem Stripper zurückgewonnen werden. Grundsätzlich könnte auch ein Sumpfprodukt erzeugt werden, das nur noch Spuren von Methanol enthält und direkt einer biologischen Reinigung zugeführt werden könnte. Da die Aufreinigungseinrichtung 70 jedoch bereits einen Abwasserstripper enthält, ist die dargestellte Lösung die effizientere.

Das Kopfprodukt der Kolonne 40 stellt im Wesentlichen ein Gemisch aus Methanol und DME dar, welches über Leitung 41 einer Kolonne 50 zugeführt wird. Die Kolonne 50 ist mit der Trenneinrichtung 20 vorteilhafterweise so verbunden, dass das im Sumpf der Trennvorrichtung 50 anfallende Methanol der Trennvorrichtung 20 als Rückfluss zugeführt wird und so über Leitung 21 ebenfalls der Umsetzung zu Dimethylether in Reaktor 30 zugeführt werden kann. Über Kopf wird aus der Kolonne 50 über Leitung 51 gereinigter Dimethylether abgeführt, der bei ca. 10 bar und einer Temperatur von ca. 50 °C gewonnen wird. Er kann so direkt dem Reaktor 60 zur Umsetzung von DME zu Olefinen zugeführt werden. Die Reinheit des Dimethylethers beträgt 95 bis 100 Gew.-% und ist, insbesondere hinsichtlich des Methanolanteils, über das Rückflussverhältnis der Kolonne 50 einstellbar. Zu beachten ist dabei, dass eine Minimierung des Methanolanteils (< 0,5 Gew.-%) ein überproportional steigendes Rückflussverhältnis erfordert, wodurch der Dampfverbrauch der Kolonne stark steigen würde. Ein Anteil von 0,1 bis 7 Gew.-%, bevorzugt 0,5 bis 1.5 Gew.-% stellt einen sinnvollen Kompromiss zwischen Dampfverbrauch und Methanol-"Schlupf" dar. Der Dimethylether erhält weiterhin zu geringen Anteilen, nämlich 0.5 - 2 Gew.-Gew.-% C₄- und C₅-Kohlenwasserstoffe und weitere Oxygenate sowie Spuren von Wasser, die im Wesentlichen über das Methanol in den DME-Synthesekreislauf gelangt sind. Diese Beimengen beeinträchtigen die Qualität des Dimethylethers nicht, weil sie erfindungsgemäß vor dem Reaktor 60 über das Recyclegas sowieso hinzugefügt werden.

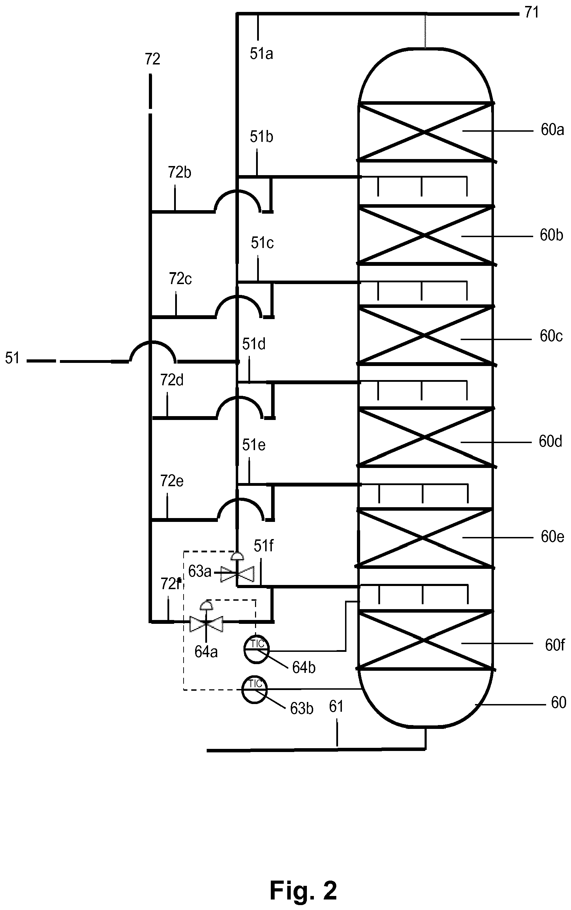

Der in Fig. 2 dargestellte Reaktor 60 zur Umsetzung von DME zu Olefinen ist als Festbettreaktor mit mehreren Katalysatorstufen 60a-60f ausgestaltet. Es empfiehlt sich, wenigstens 4, vorteilhafter wie in Fig. 2 dargestellt 6 Katalysatorstufen zu verwenden. Die Beschickung mit Dimethylether erfolgt durch eine Aufteilung des Zuflussstroms 51 in die einzelnen Ströme 51a-51f. Gleichzeitig wird über Leitungen 71 und 72 Recyclegas zum Reaktor 60 geführt und dort über Leitung 71 in die erste Katalysatorstufe 60a und über die Leitungen 72b-f in die Katalysatorstufen 60b-60f eindosiert. Das Recyclegas wird in der Aufreinigungseinrichtung 70 erzeugt und enthält olefinische, paraffinische und aromatische Kohlenwasserstoffe, bevorzugt mit C₂- sowie C₄- bis C₈-Kohlenstofffraktionen. Das Gas enthält auch kleine Anteile an Methanol, die über die Aufbereitung des Recyclegases und die Regeneration des Waschmethanols in das Recyclegas gelangen.

Das über Leitung 71 zur ersten Katalysatorstufe des Reaktors 60 geführte Recyclegas wird in einer zur Aufbereitungseinheit 70 zugehörigen Unterfeuerungseinheit auf die Eintrittstemperatur vorerhitzt. Sodann wird über Leitung 51 eine auf 250 - 350 °C vorerhitzte anteilige Menge Dimethylether zugemischt, sodass die Mischtemperatur des in den Reaktor 60 eintretenden kombinierten Gasstroms z.B. 474 °C beträgt.

Die Zuführung von Dimethylether über die Leitungen 51b, c und folgende sowie die Zuführung des Recyclegases über die Leitungen 72b, c und folgende auf die Katalysatorstufen 60 a und folgende des Reaktors 60 kann auf unterschiedliche Weise erfolgen. Denkbar wäre, Dimethylether und Recyclegas durch zwei getrennte Zufuhrleitungen und Verteiler auf die jeweilige Katalysatorstufe aufzugeben. Eine besonders einfache Ausgestaltung sieht vor, DME und Recyclegas vorzumischen und über einen gemeinsamen Verteiler auf die Katalysatorstufe aufzugeben. Einer der beiden Ströme, vorzugsweise das Recyclegas, kann vorgewärmt und somit eine Feinregelung der Temperaturprofile in den Katalysatorbetten erreicht werden. Das Einsatzgas wird über einen nicht dargestellten Gasverteiler gängiger Bauart über den Querschnitt des Reaktors 60 verteilt. Die einzelnen Katalysatorstufen sind hintereinander geschaltet, was durch die Verbindungen 62a, 62b und 62c angezeigt werden soll. Durch die Vermischung des kalten Einsatzgases mit dem aus der vorhergehenden Katalysatorstufe austretenden heißen Reaktionsgas wird letzteres gekühlt und kann so in der folgenden Reaktionsstufe mit dem beigemischten Dimethylether im gewünschten Temperaturbereich reagieren.

Grundsätzlich ist es möglich, Recyclegas mit unterschiedlichen Gaszusammensetzungen auf die einzelnen Katalysatorstufen aufzugeben, wobei es sich insbesondere empfiehlt, als einfache Möglichkeit der Ansteuerung Recyclegas mit einer abweichenden Zusammensetzung auf die erste Katalysatorstufe aufzugeben, während alle nachfolgenden Katalysatorstufen mit Recyclegas gleicher Zusammensetzung beschickt werden. Bei einer solchen Ausgestaltung ist es insbesondere sinnvoll, den gesamten Wasserdampf bereits auf die erste Katalysatorstufe, d.h. über Leitung 71 direkt zum Reaktor 60 aufzugeben und auf die Katalysatorstufen 2 bis n eine Fraktion mit einem minimalen Methanolbestandteil zu geben, da so die hohe Exothermie als Folge der Umsetzung von Methanol vermieden werden kann. In der ersten Katalysatorstufe kann die Exothermie hingegen zum "Anspringen" der Reaktion beitragen bzw. eine möglichst niedrigere Eintrittstemperatur erlauben, so dass sich die Aufgabe eines relativ Methanol-reichen Stroms anbieten könnte.

Günstig ist es im Rahmen der Erfindung auch, Recyclegas mit einem besonders hohen Olefinanteil, insbesondere mit Kettenlängen C₄ bis C₈ zu verwenden. Ob auch C₂-Kohlenwasserstoffe im Recyclegas zum Reaktor 60 zurückgeführt werden, richtet sich danach, ob in der Anlage Ethylen als Nebenprodukt gewonnen werden soll oder die Propylenausbeute maximiert werden soll. Da diese Olefine in dem Reaktor 60 in einer endothermen Reaktion zur Propylen (Ethylen) reagieren, dienen sie somit gleichzeitig zur Kühlung des Systems und erhöhen die Ausbeute an Zielprodukt.

Die Zusammensetzung des Recyclegases wird in der Aufbereitungseinrichtung 70 eingestellt. Eine für die Olefingewinnung vorteilhafte Ausführung dieser Aufbereitungseinrichtung 70 ist in DE 10 2011 014 892 A1 beschrieben. Grundsätzlich wird in der Aufreinigungseinrichtung 70 das gewünschte Hauptprodukt destillativ von den Nebenprodukten abgetrennt. Als Nebenprodukte werden vorzugsweise Flüssiggas- und Benzinfraktionen ausgeschleust. Als Recycleströme werden Kohlenwasserstoffströme und Prozesswasserströme bereitgestellt. Überschüssiges Prozesswasser wird ausgeschleust. Vorzugsweise wird der Produktstrom in der nicht näher dargestellten Aufreinigungseinrichtung 70 bspw. nach der DE 10 2011 014 892 A1 so aufgearbeitet, dass ein Propylenstrom mit einer Reinheit >99,5 Gew.-%, eine Fraktion an C₄-Kohlenwasserstoffen und eine Fraktion von C₅₊-Kohlenwasserstoffen abgezogen werden kann. Aus der Aufreinigungseinrichtung 70 kann auch Recyclegas gewonnen und über Leitung 71 und Leitung 72 in den Reaktor 60 geführt werden, wobei es grundsätzlich auch denkbar ist, nur eine Recyclegasleitung aus der Vorrichtung zu führen, oder Recyclegas mit mehr als zwei verschiedenen Zusammensetzungen zu verwenden, wodurch entsprechend mehr Zufuhrleitungen erforderlich wären.

Schließlich wird über Leitung 73 aus der Aufreinigungseinrichtung 70 beladenes Methanol aus der Methanolwaschkolonne abgezogen und einer Destillationsvorrichtung 80 zugeführt. Im Kopf dieser Kolonne reichern sich C₄/C₅-Kohlenwasserstoffe, Dimethylether und weitere Oxygenate an. Dieser Strom kann vorteilhaft wenigstens teilweise direkt über Leitung 83 als Recyclegas zum Reaktor 60 geführt werden oder über Leitung 82 zwecks weiterer Behandlung in die Aufreinigungseinrichtung 70 zurückgeführt werden. Vom Sumpf der Kolonne 80 wird über Leitung 81 das regenerierte Methanol abgezogen. Dieses enthält Restanteile C₄- und C₅-Kohlenwasserstoffe sowie Wasser und wird vorteilhaft in die Kolonne 40 als Rückfluß aufgegeben, wodurch Wasser aus dem System entfernt wird, die C₄/C₅-Kohlenwasserstoffe mit Dimethylether zum Reaktor 60 geführt werden und Methanol in den DME-Reaktor 30 eingespeist wird.

Anstelle der Kolonne 80 zur Regenerierung des Waschmethanols ist es auch möglich, nach dem Stand der Technik einen Flüssig-Flüssig-Extraktor einzusetzen. Dadurch wird eine wässrige Methanollösung mit relativ großem Wasseranteil erzeugt. Diese kann ebenso zur Kolonne 40 geführt und bei entsprechend höherem Dampfverbrauch verarbeitet werden.

Grundsätzlich ist es möglich, sowohl zur Umsetzung von Methanol zu DME als auch zur Umsetzung von DME zu Olefinen mehrere Reaktoren zu verwenden.

Fig. 2 zeigt noch einmal die Ausgestaltung eines erfindungsgemäßen Reaktors 60 mit 6 Katalysatorstufen. Dabei wird über Leitung 51 Dimethylether zugeführt, der über die Teilleitungen 51a-f auf die einzelnen Katalysatorstufen verteilt wird.
Die Zumischung von Recyclegas und Wasserdampf erfolgt für die erste Katalysatorstufe über die Leitung 71, während für die Stufen 2 bis 6 Recyclegas über Leitung 72 zugeführt wird. Dieses Recyclegas hat bspw. eine Temperatur von 98 °C, wobei eine Temperatur im Bereich von 40 bis 150 °C grundsätzlich geeignet ist.

Die Mischung von Dimethylether und Recyclegas ist in der letzten Stufe 60f exemplarisch dargestellt. Über einen Temperaturregler 63b und das zugeordnete Regelventil 63a wird die DME-Menge so eingestellt, dass nach dem Katalysatorbett 60f die gewünschte Solltemperatur des Austrittstroms erreicht wird. Durch das über Leitung 51f zugeführte kalte DME-Gas wird zwar bereits eine gewisse Kühlung erreicht, wenn sich dieser Strom mit dem Produktstrom aus der vorgeschalteten Katalysatorstufe 60e mischt, jedoch reicht diese Kühlung nicht aus. Daher wird über Ventil 64a zusätzlich Recyclegas zudosiert, so dass über den Temperaturregler 64b auch die gewünschte Eintrittstemperatur der Reaktionsmischung in das Katalysatorbett eingestellt werden kann.

Dieses Temperatur- und Reaktionsführungskonzept wird günstigerweise bei allen anderen Stufen, zumindest aber bei den Stufen 2 bis 6 gleichartig ausgeführt. Die Ein- und Austrittstemperaturen aus der jeweiligen Stufe sind flexibel und über das Mengenverhältnis der jeweiligen DME- und Recyclingströme einfach einstellbar. Es kann also über den gesamten Reaktor ein für eine maximale Propylenausbeute optimales Temperaturprofil eingestellt werden.

### Beispiel

Im Einzelnen kann ein wie in Fig. 2 dargestellter Reaktor mit den folgenden Einstellungen optimal betrieben werden:
Über die Katalysatorstufen 60a und 60b des Reaktors kann ein ansteigendes Temperaturniveau eingestellt und in der zweiten Katalysatorstufe die Zusatzkühlung mit Recyclegas minimiert werden. Im Vergleich zu den folgenden Katalysatorstufen ist dann mit einer etwas geringeren Propylenausbeute zu rechnen. Die Temperatur in den Katalysatorstufen 60a und 60b liegt zwischen 470 und 500 °C.

In den Katalysatorstufen 60c bis 60e findet der Hauptumsatz des Prozesses statt, der deutlich höher als in einer konventionellen Ausführung des Prozesses ist. Infolge einer Einstellung von entsprechenden Mengen Kühlgas liegt die Temperatur in den Katalysatorstufen 60c bis 60e zwischen 470 und 500 °C.

In der letzten Katalysatorstufe 60f wird ein reduzierter Umsatz an Dimethylether bzw. ein möglichst flach verlaufendes Temperaturintervall über das Katalysatorbett eingestellt. Erfindungsgemäß bewegt sich dann das Temperaturprofil in der Katalysatorstufe 60f bspw. zwischen 480 und 500 °C. Damit findet bei maximaler Temperatur und geringer Neubildung aus DME eine möglichst weitgehende Abreaktion der im Reaktionsgas befindlichen C₂- und C₄- bis C₈- Olefine zu Propylen statt.

Vergleichbare Einstellungen sind bei konventioneller Ausgestaltung des Reaktors nur eingeschränkt möglich, da die Exothermie der entsprechenden Reaktion bei der Anwesenheit von Methanol niedrigere Eintrittstemperaturen erfordert und damit eine entsprechend reduzierte Propylenausbeute einhergeht.

Unter sonst vergleichbaren Bedingungen kann bei einer DME basierten MTP-Anlage mit einer um 3 bis 4 Gew.-% größeren Propylenausbeute (bezogen auf das eingesetzte Methanol) gerechnet werden. Die Ausbeuteverbesserung wird durch eine Kombination der folgenden Maßnahmen und Effekte bewirkt:
Die geringere Wärmetönung der Reaktion bei Umsatz von Dimethylether anstelle von Methanol führt zu einer Erhöhung der Eintrittstemperaturen um 2 bis 8 °C und insgesamt zu flacheren und zu höheren Temperaturen verlagerten Wärmeprofilen innerhalb der einzelnen Katalysatorstufen. Der Reaktor kann so bei maximaler Reaktionstemperatur (d.h. rund 500 °C bei Austritt aus der jeweiligen Katalysatorstufe) betrieben werden, ohne dass es zu einer Schädigung des Katalysators kommt.

Günstig für die Reaktion von Dimethylether zu Propylen ist weiterhin der erhöhte Wasserdampfanteil innerhalb der einzelnen Katalysatorstufen. Im Standard-MTP-Verfahren entsteht Dampf durch die Reaktion von Methanol. Dies bedeutet jedoch auch, dass er erst innerhalb der jeweiligen Katalysatorstufe gebildet wird und somit der Anteil über die jeweilige Katalysatorstufe zunimmt. Im erfindungsgemäßen Verfahren wird hingegen Dampf als Bestandteil des Recyclegases vor der ersten Katalysatorstufe zugemischt, weshalb dieser Dampf bereits vor Beginn einer chemischen Reaktion vorliegt.

Weiterhin erlaubt die Verwendung von Dimethylether eine Optimierung der Umsätze innerhalb der Katalysatorstufen, ohne dass die Eintrittstemperaturen für die jeweiligen Stufen stark abgesenkt werden müssen. Es ist somit eine Temperaturführung innerhalb der Reaktionsstufen möglich, bei der die Umsetzung zu Propylen mit einer hohen Selektivität abläuft. Vorteilhaft wirkt sich weiterhin die Verwendung der letzten Katalysatorstufe als "Nachreaktions"-Stufe auf die Gesamtausbeute aus, weil so der Anteil an C₄₊-Olefinen in den Produktströmen minimiert werden, da hier höherkettige Kohlenstofffraktionen noch zu Propylen umgesetzt werden können.

Die Möglichkeit, die Reaktion bei höheren Temperaturen durchführen zu können, ermöglicht zudem eine geringere Verweilzeit. Bei den angegebenen Temperaturwerten sinkt bspw. die Verweilzeit um 10 %. Zudem ist eine geringere Verweilzeit innerhalb der einzelnen Reaktionsstufen jetzt auch dadurch möglich, dass aufgrund der guten Regelungsmöglichkeiten der Bedingungen in jeder einzelnen Katalysatorstufe der Umsatz leicht auf Stufen mit einem weniger gealterten Katalysator verteilt werden kann und somit die auf den gesamten Reaktor bezogenen Reserven an Katalysator ohne Durchbruch von größeren Mengen an Dimethylether und Methanol ausgenutzt werden können.

### Bezugszeichenliste

- 10: Rektifikationskolonne
- 11-13: Leitungen
- 14: Rektifikationskolonne
- 15-17: Leitungen
- 20: Trenneinrichtung
- 21, 22: Leitungen
- 30: Reaktor
- 31: Leitung
- 40: Rektifikationskolonne
- 41: Leitung
- 50: Rektifikationskolonne
- 51, 52: Leitungen
- 60: Reaktor
- 60a-60f: Katalysatorstufen
- 61: Leitung
- 62a-62c: Verbindung zwischen Katalysatorstufen
- 63a, 64a: Regelventil
- 63b, 64b: Temperaturregler
- 70: Aufreinigungseinrichtung
- 71-73: Leitungen
- 80: Rektifikationskolonne
- 81, 82, 83: Leitungen

## Patentansprüche

1. Verfahren zur Herstellung von Olefinen aus Dimethylether, wobei gasförmiger Dimethylether in einer Reinheit von 70 - 100 Gew.-%, bevorzugt 85 - 99 Gew.-% zusammen mit einem ersten Recyclegas, welches olefinische, paraffinische und/oder aromatische Kohlenwasserstoffe enthält, sowie Wasserdampf auf eine erste Katalysatorstufe eines Reaktors aufgegeben wird und wobei gasförmiger Dimethylether in einer Reinheit von 70 - 100 Gew.-%, bevorzugt 85 - 99 Gew.-%, zusammen mit einem ersten Recyclegas, welches olefinische, paraffinische und/oder aromatische Kohlenwasserstoffe enthält, auf wenigstens eine nachgeschaltete Katalysatorstufe gegeben wird, wobei diese nachgeschaltete Katalysatorstufe zusätzlich mit Produktgas aus der vorgeschalteten Katalysatorstufe gespeist wird, **dadurch gekennzeichnet, dass** das erste Recyclegas innerhalb einer Aufreinigung, der die aus Dimethylether gewonnen Olefine unterzogen werden, gewonnen wird; und
dass das in einer Aufbereitungseinheit erzeugte mit Methanol beladene Waschmittel in einer Destillationseinheit aufbereitet und das Kopfprodukt dieser Destillationseinheit wenigstens teilweise als zweites Recyclegas direkt zu einer der ersten katalysatorstufe nachgeschalteten Katalysatorstufe geführt wird; und
dass das Verhältnis von Dimethylether und Recyclegasen so eingestellt wird, dass die Eintrittstemperatur in die Katalysatorstufe zwischen 440 und 490 °C und die Austrittstemperatur aus der Katalysatorstufe zwischen 460 und 520 °C liegt.

2. Verfahren nach Anspruch 1, **dadurch gekennzeichnet, dass** das Verhältnis der auf die erste Katalysatorstufe aufgegebenen Gasströme so eingestellt wird, dass der Anteil des Dimethylethers im Verhältnis zum Recyclegas bei Eintritt in die Katalysatorstufe bei 2 bis 20 Gew.-%, bevorzugt 5-15 Gew.-%, und der Anteil des Wasserdampfes am Gesamtstrom bei Eintritt in die Katalysatorstufe maximal 60, bevorzugt 50 Gew.-%, beträgt und/oder dass das Verhältnis der auf eine nachgeschaltete Katalysatorstufe aufgegebenen Gasströme so eingestellt wird, dass der Anteil des Recyclegases im Verhältnis zum Dimethylether bei Eintritt in die Katalysatorstufe 10-200 Gew.-%,, bevorzugt 15-50 Gew.-%, beträgt.

3. Verfahren nach einem der vorhergehenden Ansprüche, **dadurch gekennzeichnet, dass** eine Mischung aus Dimethylether und Recyclegas auf wenigstens zwei Katalysatorstufen gegeben wird, wobei das jeweils aufgegebene Recyclegas eine unterschiedliche Zusammensetzung aufweist.

4. Verfahren nach einem der vorhergehenden Ansprüche, **dadurch gekennzeichnet, dass** der Druck am Eintritt des Reaktors 0.8-5.0 bar (a), vorzugsweise 1.5-3.0 bar (a) beträgt.

5. Verfahren nach einem der vorgehenden Ansprüche, **dadurch gekennzeichnet, dass** das Recyclegas einen Anteil von 10-70 Gew.-%, bevorzugt 20-40% Gew-%, an C₂- und/oder C₄-C₈ -Olefinen Gew.-% enthält.

6. Anlage zur Herstellung von Olefinen aus Dimethylether, zur Durchführung eines Verfahrens nach einem der vorhergehenden Ansprüche, mit einem Reaktor , welcher wenigstens zwei hintereinandergeschaltete Katalysatorstufen zur Umsetzung von Dimethylether zu Olefinen aufweist, **dadurch gekennzeichnet, dass** in wenigstens eine Katalysatorstufe wenigstens eine Zufuhrleitung zur Zumischung von Dimethylether, Recyclegasen und/oder Wasserdampf mündet, dass in wenigstens eine nachgeschaltete, mit der ersten Stufe verbundene Katalysatorstufe eine Leitung zur Einspeisung von Recyclegas mündet, dass ein Recyclegas jeweils olefinische, parafinische und/oder aromatische Kohlenwasserstoffe enthält und dass wenigstens eine Steuer- oder Regelvorrichtung zur Dosierung der Recyclegase im Verhältnis zu dem eingesetzten Dimethylether vorgesehen ist und dass die Zufuhrleitungen für die Recyclegase mit einer Aufreinigungseinrichtung zur Aufbereitung der Olefine bzw. einer Destillationseinheit zur Regeneration von Methanol-Waschmittel verbunden sind und dass die Regelvorrichtung so ausgestaltet ist, dass die Temperatur des Eintrittsstroms in die jeweilige Katalysatorstufe und des Austrittsstroms der jeweiligen Katalysatorstufe gemessen und als Regelgröße verwendet wird.

## Claims

1. Process for the producing of olefins from dimethyl ether, wherein gaseous dimethyl ether in a purity of 70 - 100 wt.-%, preferably 85 - 99 wt.-%, together with a first recycle gas which contains olefinic, paraffinic and/or aromatic hydrocarbons and water vapor is fed to a first catalyst stage of a reactor, wherein gaseous dimethyl ether in a purity of 70 - 100 wt.-%, preferably 85 - 99 wt.-% together with a first recycle gas which contains olefinic, paraffinic and/or aromatic hydrocarbons, is fed to at least one downstream catalyst stage, whereby this downstream catalyst stage additionally being fed with product gas from the upstream catalyst stage, **characterized in that** the first recycle gas is recovered within a purification to which the olefins obtained from dimethyl ether are subjected, and that a washing agent loaded with methanol, which is produced in a processing unit, is processed in a distillation unit, and a top product of this distillation unit is fed at least partially as a second recycle gas directly to a catalyst stage which is connected downstream of the first catalyst stage; and that the ratio of dimethyl ether and recycle gases is adjusted such that the inlet temperature into the catalyst stage is between 440 and 490 °C and the outlet temperature from the catalyst stage between 460 and 520 °C.

2. Process according to claim 1, **characterized in that** the ratio of the gas streams fed to the first catalyst stage is adjusted such that the amount of dimethyl ether in relation to the recycle gas on entry into the catalyst stage is 2 to 20 wt.-%, preferably 5-15 wt.-%, and the amount of water vapor in the entire stream on entry into the catalyst stage is maximal 60 wt.-%, preferably 50 wt.-%, and/or that the ratio of the gas streams fed to a downstream catalyst stage is adjusted such that the amount of recycled gas in relation to dimethyl ether on entry into the catalyst stage is 10-200 wt.-%, preferably 15-50 wt.-%.

3. Process according to any of the preceding claims, **characterized in that** a mixture of dimethyl ether and recycle gas is added to at least two catalyst stages, whereby the respective added recycle gas has a different composition each.

4. Process according to any of the preceding claims, **characterized in that** the pressure at the inlet of the reactor is 0.8-5.0 bar (a), preferably 1.5-3.0 bar (a).

5. Process according to any of the preceding claims, **characterized in that** the recycle gas contains an amount of 10-70 wt.-%, preferably 20-40 wt.% by weight of C2 and/or C4-C8 -olefins.

6. Plant for producing olefins from dimethyl ether for carrying out a process according to one of the preceding claims with a reactor which includes at least two catalyst series connected stages for the conversion of dimethyl ether to olefins, **characterized in that** at least one supply conduit for admixing dimethyl ether, recycle gas and/or water vapor opens into at least one catalyst stage, that a supply conduit for recycle gas opens into at least one downstream catalyst stage which is connected to the first stage, that each recycle gas contains olefinic, paraffinic and/or aromatic hydrocarbons and that at least one control or regulating device is provided for dosing the recycle gases in relation to the used dimethyl ether, and that the supply conduits for the recycle gas are connected with a purification means for processing the olefins or with a distillation unit for the regenerating methanol washing agent detergent, and that the control device is designed such that the temperatures of the inlet stream into the respective catalyst stage and of the outlet stream of the respective catalyst stage is measured and used as a control variable.

## Revendications

1. Procédé de fabrication d'oléfines à partir d'éther diméthylique, consistant à introduire de l'éther diméthylique gazeux ayant une pureté comprise entre 70 et 100 % en poids, de préférence entre 85 et 99 % en poids, ensemble avec un premier gaz de recyclage contenant des hydrocarbures oléfiniques, paraffiniques et/ou aromatiques ainsi qu'avec de la vapeur d'eau, dans un premier étage catalytique d'un réacteur, et à introduire de l'éther diméthylique gazeux ayant une pureté comprise entre 70 et 100 % en poids, de préférence entre 85 et 99 % en poids, ensemble avec un premier gaz de recyclage contenant des hydrocarbures oléfiniques, paraffiniques et/ou aromatiques, dans au moins un étage catalytique situé en aval, ledit étage catalytique situé en aval étant alimenté, en outre, avec du gaz produit par l'étage catalytique situé en amont, **caractérisé en ce que** premier gaz de recyclage est issu d'une purification à laquelle on soumet les oléfines obtenus à partir d'éther diméthylique; et
**que** l'agent de lavage, généré dans une unité de traitement et chargé de méthanol, est traité dans une unité de distillation pour introduire le produit de tête de ladite unité de distillation au moins partiellement, en tant que deuxième gaz de recyclage, directement dans un étage catalytique situé en aval du premier étage catalytique; et
**que** le rapport entre l'éther diméthylique et les gaz de recyclage est ajusté de manière à ce que la température d'entrée dans l'étage catalytique soit comprise entre 440 et 490 °C et la température de sortie de l'étage catalytique soit comprise entre 460 et 520 °C.

2. Procédé selon la revendication 1, **caractérisé en ce que** le rapport entre des flux de gaz introduits dans le premier étage catalytique est ajusté de manière à ce que la proportion de l'éther diméthylique, rapportée au gaz de recyclage, est comprise entre 2 et 20 % en poids, de préférence entre 5 et 15 % en poids, à l'entrée dudit étage catalytique, et la proportion de vapeur d'eau dans le flux global soit inférieure ou égale à 60, de préférence égale à 50 % en poids, à l'entrée dudit étage catalytique et/ou que le rapport entre les flux de gaz introduits dans un étage catalytique situé en aval est ajusté de manière à ce que la proportion du gaz recyclé, rapportée à l'éther diméthylique, soit comprise entre 10 et 200 % en poids, de préférence entre 15 et 50 % en poids, à l'entrée dudit étage catalytique.

3. Procédé selon l'une des revendications précédentes, **caractérisé en ce que** l'on introduit un mélange d'éther diméthylique et de gaz recyclé dans au moins deux étages catalytiques, le gaz recyclé que l'on introduit ayant dans chacun des cas une composition différente.

4. Procédé selon l'une des revendications précédentes, **caractérisé en ce que** la pression à l'entrée du réacteur est comprise entre 0,8 et 5,0 bar (a), de préférence entre 1,5 et 3,0 bar (a).

5. Procédé selon l'une des revendications précédentes, **caractérisé en ce que** le gaz recyclé contient une proportion d'oléfines en C₂ et/ou en C₄-C₈ comprise entre 10 et 70 % en poids, de préférence entre 20 et 40 % en poids.

6. Installation de fabrication d'oléfines à partir d'éther diméthylique, permettant de mettre en oeuvre un procédé selon l'une des revendications précédentes et comportant un réacteur pourvu d'au moins deux étages catalytiques successifs destinés à convertir de l'éther diméthylique en oléfines, **caractérisée en ce qu'**au moins une ligne d'arrivée, destinée à ajouter de l'éther diméthylique, des gaz de recyclage et/ou de la vapeur d'eau en les mélangeant, débouche dans au moins un étage catalytique, qu'une ligne destinée à l'alimentation en gaz de recyclage débouche dans au moins un étage catalytique qui est situé en aval et qui est relié au premier étage, qu'un gaz de recyclage concerné contient des hydrocarbures oléfiniques, paraffiniques et/ou aromatiques, et qu'elle comporte au moins un dispositif de commande ou de réglage permettant le dosage des gaz de recyclage par rapport à l'éther diméthylique mis en oeuvre, et que les lignes d'arrivée pour des gaz de recyclage sont reliées à un dispositif de purification destiné à traiter les oléfines ou à un dispositif de distillation destiné à la régénération d'agents de lavage au méthanol, et que le dispositif de réglage est configuré de manière à mesurer la température du flux d'entrée dans l'étage catalytique concerné et du flux de sortie de l'étage catalytique concerné pour l'utiliser comme paramètre de réglage.
